(19) Europäisches Patentamt / European Patent Office / Office européen des brevets

(11) EP 2 735 339 B1

(12) **EUROPEAN PATENT SPECIFICATION**

(45) Date of publication and mention of the grant of the patent: **22.10.2014 Bulletin 2014/43**

(51) Int Cl.: *A61P 33/02* (2006.01) *A61K 36/06* (2006.01) *A61K 36/062* (2006.01)

(21) Application number: **12194343.5**

(22) Date of filing: **27.11.2012**

(54) **Extract and isolated compounds from Drechslera rostrata and/or Eurotium tonophilum for use as anti-leishmania-major agents**

Extrakt und isolierte Verbindungen aus Drechslera rostrata bzw. Eurotium tonophilum zur Verwendung als Anti-Leishmania-major-Mittel

Extrait et composés isolés à partir de Drechslera rostrata et/ou d'eurotium tonophilum pour utilisation en tant qu'agents anti-leishmania major

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.05.2014 Bulletin 2014/22**

(73) Proprietor: **King Saud University 11421 Riyadh (SA)**

(72) Inventors:
- **Dr. Amani Shafeek Awaad Riyadh 11333 (SA)**
- **Alzylaei, Hyfa Mobarak Riyadh 11333 (SA)**
- **El-Desouky Zain, Mohamed Riyadh 11333 (SA)**
- **Al-Olayan, Ebtisam Mohamed Riyadh 11333 (SA)**
- **El-Meligy, Reham Moustafa Riyadh 11333 (SA)**
- **Alqausomy, Saleh Ibraheem Riyadh 11333 (SA)**

(74) Representative: **Goddar, Heinz J.**
**Boehmert & Boehmert Anwaltspartnerschaft mbB Patentanwälte Rechtsanwälte Pettenkoferstrasse 20-22 80336 München (DE)**

(56) References cited:
**US-A1- 2011 178 169 US-B1- 6 265 347**

- **JIANGTAO GAO ET AL: "Antimicrobial and antiprotozoal activities of secondary metabolites from the fungus", MEDICINAL CHEMISTRY RESEARCH, BIRKHÄUSER-VERLAG, BO, vol. 21, no. 10, 11 November 2011 (2011-11-11), pages 3080-3086, XP035103222, ISSN: 1554-8120, DOI: 10.1007/S00044-011-9798-7**
- **ROSA LUIZ H ET AL: "LEISHMANICIDAL, TRYPANOCIDAL, AND CYTOTOXIC ACTIVITIES OF ENDOPHYTIC FUNGI ASSOCIATED WITH BIOACTIVE PLANTS IN BRAZIL", BRAZILIAN JOURNAL OF MICROBIOLOGY, SOCIEDADE BRASILEIRA DE MICROBIOLOGIA, SAO PAULO, BR, vol. 41, no. 2, 1 April 2010 (2010-04-01), pages 420-430, XP009166460, ISSN: 1517-8382, DOI: 10.1590/S1517-83822010000200024**
- **VALADARES DIOGO G ET AL: "Prophylactic or therapeutic administration of Agaricus blazei Murill is effective in treatment of murine visceral leishmaniasis", EXPERIMENTAL PARASITOLOGY, NEW YORK, NY, US, vol. 132, no. 2, 1 October 2012 (2012-10-01), pages 228-236, XP009166462, ISSN: 0014-4894, DOI: 10.1016/J.EXPPARA.2012.07.005 [retrieved on 2012-07-20]**
- **IARA F SANTIAGO ET AL: "Leishmanicidal and antitumoral activities of endophytic fungi associated with the Antarctic angiospermsDesv. and(Kunth) Bartl", EXTREMOPHILES ; LIFE UNDER EXTREME CONDITIONS, SPRINGER-VERLAG, TO, vol. 16, no. 1, 10 November 2011 (2011-11-10), pages 95-103, XP019991667, ISSN: 1433-4909, DOI: 10.1007/S00792-011-0409-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to extracts from *Drechslera rostrata* and/or *Eurotium tonophilum* and compounds isolated from *Drechslera rostrata* and/or *Eurotium tonophilum,* for use as anti-leishmania agents against Leishmania major.

Infectious disease leishmaniasis pose enormous global health threats. The treatment of this disease is difficult, as it requires prolonged and painful applications of toxic drugs that are poorly tolerated. Therefore, researchers are looking for new drugs and alternatives resources for the treatment of leishmaniasis.

There are two general approaches for treatment of leishmaniasis known in the art, which may be divided into synthetic drugs and natural products.

Steroidal compounds known as telocinobufagin and hellebrigenin have been isolated from skin secretions of toad *Rhinella jimi.* Both steroids demonstrated activity against *Leishmania chagasi* promastigotes. A nether complex mixture of epidioxysterols from the *Colombian Caribbean* and *Ircinia campana* displayed antileishmanial activity.

Three new disulfated meroterpenoids, ilhabelanol, ilhabrene and isoakaterpin, have been isolated from extracts of the Brazilian marine sponge *Callyspongia sp.* Only isoakaterpin inhibited the growth of Leishmania spp with an IC50 of 1.05 μM. The structures of this compounds were analysed using one- and two-dimensional NMR. Both ilhabelanol and ilhabrene have meroterpenoid carbon skeletons. Renieramycin A isolated from sponge *Neopetrosis sp* showed antileishmanial activity with $IC_{50}$ of 0.2 μgml.

One glycoprotein known as Pachymatismin was isolated from *Pachymatisma johnstonii* marine sponge and showed anti-leishmanial activity with $IC_{50}$ about 1 mg/ml. Two cyclic peroxides isolated from *Plukorfis aff. Ungulospiculatus* sponge showed anti-leishmanial activity. Medicinal plants have been used for centuries for treatment of various diseases and are very important source for drug discovery, particularly agents parasites and over 100 species of plants have been reported to be active against various of leishmania parasites.

Ramiflorines A and B were isolated from *Aspidosperma ramiflorum* and showed significant activity against *Leishmania amazonensis* with $LD_{50}$ values of 18.5 ± 6.5 μg/ml and 12.63 ± 5.52 μg/ml, respectively.

Chalcone, namely as chalcones 2'-hydroxy-3',4',6'-trimethoxychalcone, was isolated from *Piper hispidum* and showed activity against *Leishmania amazonensis* with an $IC_{50}$ of 0.8 mM. The purified guaianolide was isolated from the *Tanacetum parthenium* showed 50% inhibition for growth of *Leishmania amazonensis* with $IC_{50}$ 2.6 μg/ml.

Different concentrations of ethanolic extract of the *Echinacea purpurea prepared* showed antileishmanial effect. Glycosphingolipid (cerebroside) was isolated from *Desmodium gangeticum* and possesses significant in vitro antileishmanial activity against *Leishmania donovani.*

The alkaloidal extract of *Aspidosperma ramiflorum* was much more effective against *L. ama-zonensis* (LD50 < 47 μg/ml) than *L. braziliensis.* Two dihydrochalcones were isolated from *Piper elongatum Vahl* by activity-guided fractionation against extracellular promastigotes of *Leishmania braziliensis* in- vitro. Two (abietanes) diterpenes were isolated from *Salvia cilicica* and identified as (7-hydroxy-12-methoxy-20-nor-abieta-1,5(10),7,9,12-pentaen-6,14-dione) and (abieta-8,12-dien-11,14-dione (12-deoxy-royleanone)). They showed moderate antileishmanial activities against *L. donovani* and *L. major* (Tan *et al.,* 2002). Coronaridine as Indole alkaloid, isolated from *Peschiera australis* showed activity against *growth of Leishmania amazonensis.* Hexane, ethyl acetate and methanol extracts of *Annona muricata* were tested in vitro against *Leishmania braziliensis* and *L. panamensis.* The ethyl acetate extract was more active against Leishmanial growth than the other extracts.

Harmane, pleiocarpine and buchtienine alkaloids, isolated from the stem-bark extract of *Kopsia griffithii,* were found to have antileishmanial activity against the *L. donovani.* The flavonoid 5,7,4'-trihydroxyflavan was isolated from *Faramea guianensis* exhibited toxicity on amastigotes of *L. amazonensis.*

The most common treatment of leishmaniasis is from algae, bacteria and fungi. They appear to be an interesting source for ethnomedicinal and Phytochemical studies. The power of algal resources has been sought for thousands of years for their ability to prevent disease and work as antiviral.

Previous studies showed the presence of antileishmanial activity from algae. Five sesquiterpenes were isolated from red alga *Laurencia dendroidea* and identified as (+)-obtusane, a triquinane derivative, cartilagineol, (-)-elatol and obtusol. The last two compounds displayed *in vitro* and *in vivo* leishmanicidal activity and very low cytotoxicity.

The n-hexane and dichloromethane extracts of the red alga *Bostrychia tenella* were active against *L. amasonensis.* The Crude extracts of *Asparagopsis taxiformis* and *A. armata* showed antiprotozoal activity against *L. donovani.*

Aqueous and organic extracts of twenty-seven species of marine algae (14 species of *Rhodophyta,* 5 species of *Phaeophyta* and 8 species of *Chlorophyt*) were evaluated for their antileishmanial in- vitro activity against *Leishmania mexicana* promastigote forms. The Organic extracts from *Laurencia microcladia* (Rhodophyta), *Dictyota caribaea, Turbinaria turbinata* and *Lobophora variegata* (Phaeophyta) possessed promising in vitro activity against *L. mexicana* promastigotes.

Crude extracts of *Codium iyengarii, Ulva reticulate, Ulva rigida, Scinaia indica, Centroceras clavulatum* and *Botryocladia leptopoda* showed good in-vitro activity against leishmania.

Bacteria are naturally occurring microorganisms having various biotical activities Dragonamides A and E, and herbamide B, linear peptides isolated from *Lyngbya majuscula,* exhibited antileishmanial activity with IC50 values of 6.5, 5.1, and 5.9 μM, respectively. Amphotericin B produced by *Streptomyces nodosus* and showed activity against visceral leishmaniasis. Sinefungin, a natural nucleoside isolated from cultures of *Streptomyces incarnatus* and *Streptomyces griseolus* showed significantly suppressive against both *L. donovani* and *L. braziliensis.*

Fungi are plant-like organisms that lack chlorophyll and that include yeasts, moulds and mushrooms.

For antileishmanial activity there was some research which have been carried out in this field. Extracts of twelve fungi belonging to the genera *Alternaria, Antarctomyces, Cadophora, Davidiella, Helgardia, Herpotrichia, Microdochium, Oculimacula* and *Phaeosphaeria* were inhibited the proliferation of *L. amazonesis.* Eleven extracts of *Alternaria, Arthrinium, Cochliobolus, Colletotrichum, Penicillium, Fusarium,* and *Gibberella* showed inhibition to the growth of *L. amazonensis* with $IC_{50}$ values ranged from 4.6 to 24.4 μg/mL.

Thirty-four extracts of *Basidiomycota* fungi inhibited the activity of *L. amazonensis.* The crude extract of the endophytic fungus *Cochliobolus sp* killed about 90% of the amastigote-like forms of *Leishmania amazonensis.*

In addition, antileishmanial activity was recorded from eight compounds known as preussomerin EG1, palmarumycin CP2, palmarumycin CP17, palmarumycin CP18, and CJ-12,371, palmarumycin CP19, preussomerin EG2 and 5-methylochracin which were isolated from *Edenia* sp. all compounds inhibited the growth of amastigote forms of *Leishmania donovani.*

Altenusin, a biphenyl derivative was isolated from *Alternaria* sp was able to inhibit leishmaniasis with $IC_{50}$ value of 4.3+/-0.3.

The ethyl acetate extracts of endophytic fungi showed inhibition of *Leishmania tarentolae.*

Two new, 5-heptadeca-8'Z,11'Z,16-trienylresorcinol and 5-heptadeca-9'E,11'Z,16-trienylresorcinol, and six known 5-alkylresorcinols were isolated from the mushroom *Merulius incarnatus.* Compound -heptadeca-8'Z,1 1'Z,16-trienylresorcinol was active against leishmania, with an $IC_{50}$ value of 3.6 μg/mL.

The polyketide citrinin, isolated from *Penicillium janthinellum,* presented 100% activity against *Leishmania* sp. Hypocrellin A and hypocrellin B were isolated from the *Hypocrella bambusae.* Hypocrellin A exhibited potent antileishmanial activity, while hypocrellin B was only moderately active. Striatin A and striatin B, diterpenes isolated from Basidiomycetes fungi, were shown to be active against L. amazonensis.

Mycotoxin MT81, an anthraquinone and some of its derivatives derived from *Penicillium nigricans,* showed only moderate antileishmanial activity against *L. donovani* promastigotes (Majumder et al., Indian Journal of Experimental Biology 31:888-90, 1993, Chatterjee and Gupta, Indian Drugs. 28:224-227, 1991). Phaseolinone isolated from *Macrophomina phaseolina* showed inhibited the growth of *L. donovani.*

Antileishmanial activity against Leishmania donovani was observed for six secondary metabolites (phenol derivates) from the fungus Eurotium repens (Jiangtao Gao et al., Med. Chem. Res. (2012) 21: 3080 - 3086).

It is an object of the present invention to provide novel and efficient anti-leishmania agents against Leishmania major which overcome the drawbacks of the prior art.

This object is achieved by an extract from a fungal material of *Drechslera rostrata* and/or *Eurotium tonophilum* for use as anti-leishmania agent against Leishmania major.

Preferably, the extract is an alcoholic extract, more preferably is a methanolic extract.

Even preferred, the fungal material comprises mycelia mat of *Drechslera rostrata* and/or *Eurotium tonophilum.*

The object is further achieved by a compound isolated from an extract from the fungal material of Drechstera restroda and/or Eurotium tonophilum selected from hexan-1,2,3,4,5,6-hexol, diisooctylphthalate and/or 1,8-dihydroxy-3-methoxy-6-methyl antraquinone for use as anti-leishmania agent against Leishmania major.

A method for isolating hexan-1,2,3,4,5,6-hexol, diisooctyl-phthalate and/or 1,8-dihydroxy-3-methoxy-6-methyl antraquinone from *Drechslera rostrata* and/or *Eurotium tonophilum* comprises the steps:

a) extracting a fungal material of *Drechslera rostrata* and/or *Eurotium tonophilum* to obtain an extract;

b) concentrating the extract to obtain a concentrate;

c) chromatographing the concentrate into fractions; and

d) isolating fractionated compounds.

Preferably, the fungal material comprises mycelia mat of *Drechslera rostrata* and/or *Eurotium tonophilum.*

Even preferred, extracting is carried by using at least one organic solvent.

In a preferred embodiment, the organic solvent is selected from the group consisting of petroleum ether, heptane, hexane, ethanol, isopropanol, methanol and mixtures thereof, preferably is methanol.

Further preferred, chromatographing is carried out by means of thin layer chromatography (TLC), column

chromatography and/or gel filtration.

Preferably, the eluent used for chromatography is petroleum ether, heptane, ethanol, methanol, benzene, diethylether, chloroform, dichlormethan, water and/or mixtures thereof.

In a further preferred embodiment, the stationary phase used for chromatographing is silica gel, aluminium oxide or sephadex gel.

The object is further achieved by a pharmaceutical composition comprising the inventive extract and/or at least one of the inventive compounds.

Finally, the object is achieved by the inventive pharmaceutical composition, formulated for oral administration.

Surprisingly, it was found that the inventive extracts from *Drechslera rostrata* and/or *Eurotium tonophilum* and the compounds isolated thereof solve the problem underlying the present invention by providing anti-leishmania agents obtainable from natural materials exhibiting good anti-leishmania activity against Leishmania major. It was further surprisingly found that the inventive extracts and compounds can be used as anti-leishmania agents against Leishmania major without negative impact on liver and kidney functions. Finally, it was found that the inventive extracts and compounds can be isolated from the stated natural material in an easy and cost efficient way.

In accordance with the solutions of the problem and preferred embodiments, fungal alcohol extracts of *Drechslera rostrata* and *Eurotium tonophilum* were found to review significant in-view and in-vitro anti-leishmania activities against *Leishmania major* with $IC_{50}$ of 28.8 and 28.2 μg/ml, for *D. rostrata* and *E. tonophilum* respectively. Three compounds were isolated from fungal extracts showing good activity against *Leishmania major* with $IC_{50}$ from 3.1-19.2 μg/ml. The administration of oil extracts and compounds showed very good activity in comparison with pentostam as standard drug. Complete heeling activity was obtained for the extracts and the isolated compounds after three weeks while pentosam did not show complete heeling after 28 days. No side effects were recorded on liver and kidney functions before or after infection and treatment with *Leishmania major.*

"Extracting" for preparing the inventive extract or in the first step of the method, can comprise only one or more than one consecutive extraction steps. In the latter case, different solvents or solvent mixtures, in particular solvents or solvent mixtures having a significantly different polarity, can be used in the various steps.

In the same way, "chromatographing" can comprise only one chromatographic separation as well as more than one consecutive chromatographic separation steps. Particularly, different eluents and/or stationary phases can be used in each chromatographic step. Further, in case that two or more chromatographic separation steps are carried out, each step can be a different chromatographic method, for example in the first step a mixture (concentrate) is separated by column chromatography and a single fraction obtained in the first step is subsequently separated by thin layer chromatography.

The term "pharmaceutical composition", as used herein, is intended to comprise at least one pharmaceutical reactive extract of the present invention and/or at least one of the isolated compounds of the present invention and/or corresponding salts and/or pharmaceutical active derivatives thereof.

The pharmaceutical composition can be, for example, in a liquid form, e.g. a solution, sirup, elixir, emulsion and suspension, or in a solid form, e.g. a capsule, caplet, tablet, pill, powder and suppository. Granules, semi-solid forms and gel caps are also considered. In case that the pharmaceutical composition is a liquid or powder, dosage unit optionally is to be measured, e.g. in the dosage unit of a tea spoon. In addition to one of the extracts or the isolated compounds, the pharmaceutical composition can comprise, for example, flavoring agents, sweeteners, dyes, stabilizers, diluents, suspending agents, granulating agents, lubricants, binders and disintegrating agents. The table, for example, can be coated. All of the formulations mentioned can be intended for immediate-release, timed release and sustained release.

All components of the pharmaceutical composition have to be pharmaceutically acceptable. The term "pharmaceutically acceptable" means at least non-toxic. The therapeutically active compound should preferably be present in the above mentioned pharmaceutical composition in a concentration of about 0.1 to 99.5% by weight, preferably of about 0.5 to 95% by weight of the total mixture.

The above mentioned pharmaceutical composition can further contain other pharmaceutical active compounds in addition to the active extracts and/or compounds according to the invention.

Additional features and advantages of the present invention will become apparent in the following detailed description on the basis of the examples.

## Examples

### 1.) Materials and Methods

### Fungal Material

The investigated fungi, *Drechslera rostrata* (DSM 62596) and *Eurotium tonophilum* (ATCC 16440) were obtained from DSMZ (German Collection of Microorganisms and Cell Cultures). Direct inoculation method was used for sampling

and isolation of fungal isolates.

For isolation, culturing, maintenance of stock cultures, and experimental studies the following range of media was used: Czapek-Dox Agar, Malt Extract Agar (Malt extract) Potato Dextrose Agar and Yeast Extract Sucrose Agar. The liquid medium was prepared by using the same ingredients without agar.

**Apparatus**

For identification of the isolated compounds, melting points were determined on a Kofler hot-stage apparatus and are uncorrected; mass spectra (Electrospray negative ion) sample dissolved in acetonitrile on an Micromass Quattro spectrometer. $^{1}H$ and $^{13}C$ NM spectra, using external electronic referencing through the deuterium resonance frequency of the solvent, were determined at 600.17 or 150.91 MHz, respectively with a JEOL ECA 600 spectrometer, fitted with an auto 5mm X/H probe. Carbon atom types were established in the $^{13}$CNMR spectrum by employing a combination of broad- and proton-decoupled and distortionless enhancement by polarization transfer (DEPT) experiments with 64 K data points over a spectrum width of 17,605.6 Hz. [$^{1}J_{C\text{-}H}$] and [$^{2}J_{C\text{-}H}$] and [$^{3}J_{C\text{-}H}$]. $^{1}H$-$^{13}C$ correlations were established by using HMQC and HMBC pulse sequences respectively. $^{1}H$-$^{1}H$ correlations were by double quantum filtered COSY.

**Extraction and isolation**

The mycelia mat (700 g) of each fungi (*Drechslera rostrata* and *Eurotium tonophilum*) was harvested, washed with distilled water, then extracted by refluxing in boiled methanol (2 liter) for 2 hours and then filtered off. The mycelia residues were re-extracted again for three times. The combined filtrates were concentrated under reduced pressure at temperature not exceeding 35°C. The obtained residues (67 & 40 g for *Drechslera rostrata* and *Eurotium tonophilum* respectively) were kept for investigation and symbolized as RI & R2.

The total extracts of *Drechslera rostrata* (30 g) and total extracts of *Eurotium tonophilum* (27 g) were dissolved separately in methanol and applied on the top of column (150×5 cm) packed with sephadex LH-20 100 g. Elution was carried out with methanol. 300 fractions were collected (100 ml each). Similar fractions (according to colour and number of spots) were collected together and concentrated. As previously described, two fractions were obtained from each column.

For further purification the two fractions of each fungus were separately re-uploaded on the top of several columns packed with silica gel and eluted with chloroform methanol in gradual elution from which three compounds were isolated.

**2.) Biological Activities**

**1) Anti Leishmanial I activity**

**Parasite:** Promastigotes of the reference strain of *Leishmania major* were grown in RPMI-1640 medium supplemented with 10.000 Unites penicillin 10 μg of treptomycin/ml and 10 % heat inactivated fetal calf serum in 50 ml Nunc flasks at 26 °C in a dark environment. All experiments were performed with parasite cultures in the stationary phase by allowing them to grow for seven days.

**Maintaining parasite virulence:** The parasite virulence was maintained by serial inoculation of female hamsters (Syrian or Chinese female hamsters were provided by Girls collage of science, Zoology department, King Saud University, Riyadh, KSA) with *Leishmania major.* The right foot pad of the animal was inoculated with $10^{7}$ of stationary phase (8 days old) *Leishmania major* suspended in 0.1 ml RPMI medium. The Hamsters were kept at the animal house for 3-4 weeks. Foot pad with lesion that occurred after inoculation of *Leishmania major* was then homogenized after sacrificing the animal.

**Isolation of *Leishmania major:*** The animal was suffocated by using $CO_2$ in a special container. The infected foot pad was then cleaned by using 70 % Ethanol before chopping it off. In a sterilized culture dish the foot pad was sprayed with 70 % Ethanol and moved to the Tissue culture laboratory hood. The foot pad was cleaned of skin, hair and nails by using a sterile scalpel, forceps and scissors. The flesh was moved into the homogenizing apparatus, placed in ice and then 10 ml of complete RPMI1640 medium was added. The medium was mixed probably with the flesh, which was smashed by continuous movement of the apparatus. The supernatant was transferred into 15 ml tube and then spun again at 500 rpm for 5 min to sediment the remaining flesh and cells. The pellet was discarded and the supernatant was poured into a new 15 ml tube. The supernatant was transferred into 15 ml tube and spun at 2500 rpm for 15 min to sediment the parasite. In order to wash the isolated parasites, 10 ml of RPMI 1640 medium was added to the parasite pellet. Cells were re-suspended by gentle vortex and the tubes were spun at 2500 rpm for 15 min. The supernatant was discarded and then 5 ml of RPMI1640 medium was added to the tube. After vortixing the tubes gently, the mix was poured into two tissue culture flasks containing a previously prepared RPMI-1640 medium, and incubated at 26 °C for

three days.

**a) *In vitro* evaluation for activates of extracts and isolated compounds by MTT assay:**

Promastigotes were resuspended in fresh medium to achieve $10^6$ parasites/ml and were seeded in 96-well Nunc culture plate, each well containing 100 μl medium with different concentrations of total extracts for both fungi (*Drechslera rostrata* and *Eurotium tonophilum*) in addition to the four isolated compounds. Control wells received only culture medium. The plates were incubated in 26 °C for 72 hours and Promastigotes viability was evaluated by the quantitative colorimetric MTT assay. The alteration of MTT to the formazan product by the mitochondrial electron transport chain is an indicator of cell viability, and a decrease in the amount of MTT converted indicated toxicity to the cell.

Briefly, the MTT labeling reagent was added to each well to the final concentration of 0.25 mg/ml. After incubation for 3 hours at 26 °C, DMSO was added to dissolve the formazan crystals and obtain a homogeneous blue solution suitable for measurement of the absorbance with an enzyme-linked immunosorbent assay plate reader (wave length 492).

Percent growth inhibition of the parasite was calculated by the following formula:

$$\% \text{ of inhibition} = \frac{(\text{OD control} - \text{OD drugs})}{\text{OD control}} \times 100$$

The concentration inhibiting 50 % of the parasite growth (IC50) was calculated after evaluating percent growth inhibition at different concentrations. Reference compound was Pentostam.

Stock solutions of the total extracts , compounds, plus control drugs, were prepared at a concentration of 20 mg mL-1 in DMSO (Sigma, UK), and diluted to appropriate concentrations prior to assays. IC50 values were calculated with MSXLFIT (IDBS, UK) Djalma et al., J. Braz. Chem. Soc. Vol. 20, No. 4, 644-651, (2009).

*Cytotoxicity assays* 96-well plates were seeded with KB cells at 4 × 104 $mL^{-1}$ (100 μL *per* well). Total fungal extracts (*Drechslera rostrata* and *Eurotium tonophilum*) at 300, 30, 3 and 0.3 μg $mL^{-1}$ were added in fresh overlay after 24 h, in triplicate at each concentration. Plates were incubated for 72 h at 37 °C in a 5 % $CO_2$-air mixture the isolated compounds were given in different concentration (3, 2 and 0.2 μg $mL^{-1}$). At 72 h Alamar Blue was added to the plates. Plates were read after 4-5 h on a Gemini fluorescent plate reader (Sofimax Pro. 3.1.1, Molecular Devices, UK) at EX/EM 530/585 nm with a filter cut-off at 550 nm. IC50 values were calculated against the blanks and control samples.

**b) *in vivo* evaluation for activates of extracts and isolated compounds on infection:**

*Balb/c* mice (45-50 g) males were provided by Girls collage of science, Zoology department, King Saud University, Riyadh, KSA). The animals were housed in standard plastic cages and were maintained on standard feed and water *ad libitum.*

Each mouse was inoculated in the lumbar area with $10^7$ of stationary phase *Leishmania major* suspended in 0.1 ml RPMI medium. The mice were kept at the animal house for 3-4 weeks till the lesion was observed. Extracts were administrated orally (400 mg/kg mouse body weight) daily until complete recovery from the lesion. Liver functions tests and histopathological examination of organs (liver, spleen and kidney) were done at the end of study.

**II) Pharmacological Studies:**

**Preparation of fungal extract:** The ethanol extract of each fungi under investigation *(Drechslera rostrata* and *Eurotium tonophilum*) previously prepared, was suspended individually in sterile normal saline (0.9 % NaCl) with the aid of few drops of tween 80 immediately before use. The concentration of the tested extract was 10 %.

**Animals :** Swiss albino mice (20-25 g), male Sprague-Dawley rats (160-180 g) and healthy guinea pigs (950-1200 g) were employed in the study. The animals were left for 2 weeks at room conditions to ensure stabilization before use. They were maintained on standard pellet diet and water *ad libitum* throughout the experiment. A minimum of six animals was used in each group (n=6).

**Acute toxicity and median lethal dose ($LD_{50}$) test:** An initial test was done to determine the approximate lethal and non-lethal doses of (*Drechslera rostrata* and *Eurotium tonophilum*) extracts according to **Turner (1965).** For each extract, five groups of six mice each were used. Single oral doses of 1000, 2000, 3000, 4000 and 5000 mg $kg^{-1}$ body weight of each extract were administered to mice using intragastric tube. The control group was given an equal volume of the vehicle (1% v/v Tween 80). The animals were observed for 24 hours and the number of dead mice was recorded and used in the calculation of the acute toxicity value ($LD_{50}$). The mice were also observed for other signs of toxicity, such as, excitation, tremors, twitches, motor coordination, righting reflex and respiratory changes.

**Sub-chronic toxicity:** The effect of prolonged oral administration of the (*Drechslera rostrata* and *Eurotium*

*tonophilum*) extracts on liver and kidney functions was evaluated in rats. Twenty four male Sprague-Dawley rats were randomly divided into 4 equal groups. Rats of the 1st group were given the vehicle (1% v/v Tween 80) in a dose of 5 ml/kg b.wt and left as normal control. The 2nd, 3rd, and 4th groups of the rats were administered (*D. rostrata* and *E. tonophilum*) extracts in a dose of 200 mg $kg^{-1}$ b.wt. All medications were administered orally via the aid of an oral tube for 4 weeks. The animals were observed for signs of abnormalities throughout the experiment. Blood samples (2 mL) were collected from the retro-orbital venous plexus of each rat into clean centrifuge tubes at 4 weeks post-extracts administration. The blood samples were allowed to clot at room temperature for 30 min after which they were centrifuged at 10,000 rpm for 5 minutes. Sera were separate with Pasteur pipette into sterile serum sample tubes and used for biochemical assay.

**Measurement of liver and kidney function markers:** Liver function was evaluated by measuring the serum activity of aspartate aminotransferase (AST) and alanine aminotransferase (ALT) following the method of **Reitman and Frankel (1957)**, while the activities of alkaline phosphatase enzyme (ALP) was assayed by the method of Babson et al. Clinical chemistry 12: 482-490, (1966**)**. Serum levels of total bilirubin **(**Walter and Gerarade, Microchem. J. 15: 231, 1970**)**, total proteins **(**Henary et al., Clinical Chemistry Rpinciples and Techniques, 1974**)** and albumin **(**Doumas et al., Clin. Chim. Acta 31: 87-96, 1971**)** were also assayed. Globulin was obtained from the difference between total proteins and albumin. Serum concentrations of uric acid and urea were determined colormetrically as measures of kidney function according to **(**Morin and Prox, Am. J. Clin. Phatol, 60(5): 691, 1973**)** and **(**Wills and Savory, Annuals of Clinical and Laboratory Science 11(4), 292-299, 1981**),** respectively. Measurement of creatinine concentration in serum was carried out according to the method of Kroll et al., Clinical Chemistry, 33: 1129-1132, (1987**).**

**Results**

**3.) Chemistry results:**

Three compounds were isolated from *Drechslera rostrata* and *Eurotium tonophilum* and identified using different instrumental analysis ($^1$HNMR, $^{13}$CNMR, MASS and Milting points).

**Compound H1:** was obtained as whitish crystals (30 mg) from methanol (m.p. 165-166 °C) It was colorless under UV light, $R_f$ = 0.20 in system (Benzene : Ethyl acetate 86:14 v/v). $^1$H-NMR (DMSO-$D_6$): δ 3.57 (1H, m, H-2), 3.50 (1H, t, J=7.56, 7.56 Hz, H-5), 3.43 (1H, m, H-7), 3.35 (2H, m, H-3). $^{13}$C-NMR (DMSO-$D_6$); 70.26 (C-2), 64.37 (C-3), 71.88 (C-5), 71.88 (C-7), 70.26 (C-9), 64.37 (C-17). EI-MS m /z (% rel. int): 182.2($M^+$) 181.0 (M-1). This compound was identified as: ***Hexane-1,2,3,4,5,6-hexol***

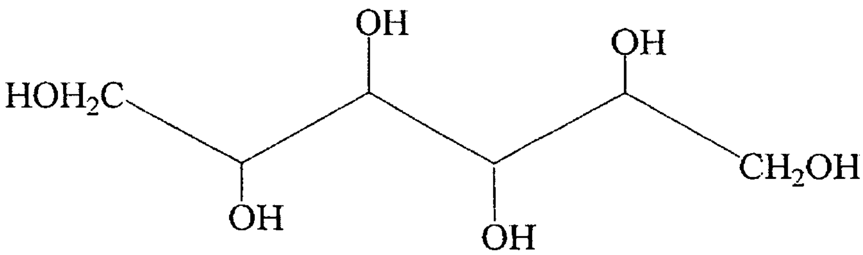

***Hexane-1,2,3,4,5,6-hexol***

**Compound H2:** obtained as colorless oily (50 mg). $R_f$ = 0.128 in system (n-hexane: benzene 30:70). Its molecular Formula was: $C_{24}H_{38}O_4$, while its Molecular Weight was 390 g/mol. This compound was identified as Diisooctylphthalate.

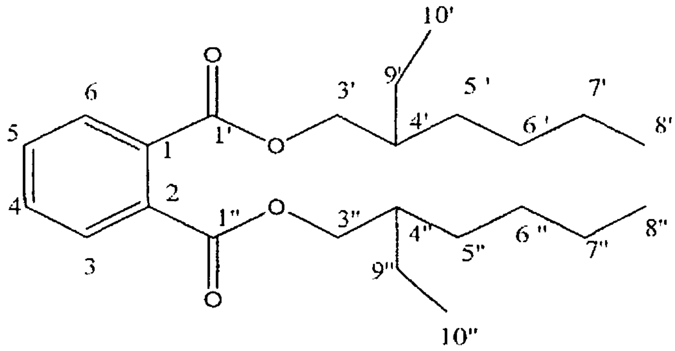

**Diisooctylphthalate**

**Compound H3:** Obtained as yellow needle crystals from $CHCl_3$, $R_f = 0.677$ in system (Benzene: $CHCl_3$ 80:20). Molecular Formula is $C_{16}H_{10}O_5$, Molecular Weight: 284 g/mol

Melting point: 207.5. The $^1$H-NMR spectrum of compound exhibited four aromatic protons [$\delta$ 6.73 (H2), 7.41 (H4), 7.55 (H5), 7.1 (H7)]. Three aliphatic methyl protons [$\delta$ 2.45 ($CH_3$)] and three aliphatic methyoxy protons [$\delta$ 3.94 ($OCH_3$)]. Two hydroxyl groups [$\delta$ 12.12 (1-OH), 12.32 (8-OH)] attaching to aromatic ring. This compound was identified as: 1,8-Dihydroxy-3-methoxy-6-methyl-anthraquinone.

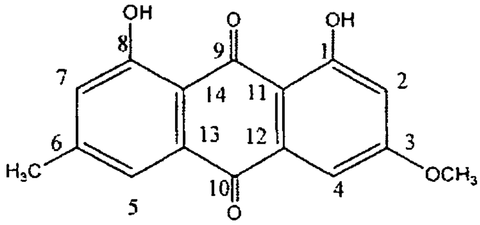

**1,8-Dihydroxy-3-methoxy-6-methyl-anthraquinone.**

**4.) Biological results:**

**a) *In- vitro* anti Leishmanial activates.** Ethanol extract of both fungi *Drechslera rostrata* and *Eurotium tonophilum* showed very good activities against *Leishmania major* ($IC_{50}$ of 28.8 and 28.2 μg/mL. For *Drechslera rostrata* and *Eurotium tonophilum* respectively).

Only two from the three isolated compounds (H 1-3) showed activities with different $IC_{50}$. The IC50 were they were; 6.5, 3.2 and 19.38 μg/mL for H1,H2 and H3 respectively

**b) *In vivo* anti Leishmanial activates:** Ethanol extract of both fungi *Drechslera rostrata* and *Eurotium tonophilum* showed very good activities against mouse infected with *Leishmania major.* The treated animals showed complete healing properties after 10 days for both extracts.

**Table 1**

Table 1:Effect of oral treatment with the fungal extracts, isolated compounds and slandered on ***L.major*** (n = 6)

| Extracts and compounds | Days of treatment Mean + SD lesion size(mm) | | | | | | | |
|---|---|---|---|---|---|---|---|---|
| | 1 | 4 | 8 | 11 | 15 | 18 | 25 | 28 |
| Untreated | 10.47±2.1 | 10.98±2.0 | 11.45±1.8 | 12.10±1.3 | 12.39±3.5 | 13.02±2.3 | 13.82±3.1 | 14.72±2.5 |
| *Drechslera rostrata extract* | 10.00±1.1 | 8.48±2.2 | 6.94±1.5 | 5.37±2.3 | 2.94±1.5 | 0.02±2.7 | zero | zero |
| *Eurotium tonophilum axtract* | 9.83±2.5 | 7.68±2.2 | 5.25±1.9 | 3.11±1.2 | 1.31±2.5 | 0.11±2.3 | zero | zero |
| H1 | 9.95±1.5 | 7.19±1.2 | 4.85±1.2 | 2.78±1.7 | 1.21+1.1 | zero | zero | zero |
| H2 | 9.13±1.2 | 6.18±1.2 | 4.15±1.2 | 1.11±1.2 | 0.21±1.5 | zero | zero | zero |
| H3 | 9.23±1.4 | 7.28±2.1 | 5.15±1.4 | 3.41±1.8 | 2.31±2.6 | zero | zero | zero |
| Pentostam | 9.56±1.3 | 8.98±2.0 | 7.45±1.8 | 6.9±1.3 | 5.39±3.1 | 4.32±2.1 | 3.30±2.1 | 2.61±2.5 |

**II) Pharmacological activities**

**Acute toxicity and median lethal dose ($LD_{50}$) test:** All the animals that received the three tested extracts in doses up to 5000 mg $kg^{-1}$ survived beyond the 24 hours of observation. On administration of any of the three extracts, no immediate behavioural changes were noted. There were no changes in the nature of stool, urine and eye color in any of the animals. All mice move and fed normally. They did not exhibit diarrhoea, haematuria, restlessness, uncoordinated muscle movements, and respiratory distress during the experimental period. The median acute toxicity value ($LD_{50}$) of the alcoholic extract of *Drechslera rostrata* and *Eurotium tonophilum* determined to be greater than 5000 mg $kg^{-1}$ body weight.

**Sub-chronic toxicity:** None of the tested extracts induced any deleterious effect on the liver and kidney functions of rats after 4 weeks of oral administration (Tables 1 - 3). Oral administration of the alcoholic extract of *Drechslera rostrata* and *Eurotium tonophilum* in a dose of 200 mg $kg^{-1}$ b.wt to rats for 4 weeks did not disturb the serum activity of AST, ALT and ALP as compared to the normal control animals (Table 3). No significant changes in the mean values of total bilirubin, total protein, albumin and globulin (Table 3) were found in serum of rats following 4 weeks of extracts administration when compared with the control. The obtained results showed insignificant effect on serum levels of urea, uric acid, and creatinine (Table 4) in the all medicated groups when compared to the control group.

No change were obtained on liver and kidney function in animal after infection with Leishmania treatment with the extracts. This indicates that both extracts are very save for treatment of *Leishmania major.*

**Table 2.** Effect of prolonged oral administration of the alcoholic extract of *Drechslera rostrata* and *Eurolium tonophilum* in a dose of 400 mg $kg^{-1}$ for 4 weeks on the serum activity of AST, ALT and ALP enzymes in rats before infection with *Leishmania major* (n = 6)

| Groups | ALT (U/L) | AST (U/L) | ALP (U/L) |
|---|---|---|---|
| Control | 76.0±6.03 | 158.3±3.81 | 391.6±11.97 |
| *D. rostrata* | 74.5±2.87 | 164.0±3.89 | 388.0±6.17 |
| *E. tonophilum* | 83.3±1.92 | 173.8+3.49 | 389.8±9.53 |
| Values represent the mean + S.E. No significant difference from control (LSD) | | | |

**Table 3.** Effect of prolonged oral administration of the alcoholic extract of *Drechslera rostrata* and *Eurotium tonophilum* in a dose of 400 mg $kg^{-1}$ for 4 weeks on the serum concentration of total bilirubin, total proteins, albumin and globulin in rats before infection with *Leishmania major* (n = 6)

| Groups | T bil (mg/dL) | T.prot m(g/dL) | Alb (mg/dL) | Glucose (mg/dL) |
|---|---|---|---|---|
| Control | 1.0±0.09 | 6.1±0.11 | 3.4±0.07 | 82.0±1.46 |
| *D. rostrata* | 1.1±0.08 | 6.3±0.16 | 3.2±0.09 | 76.5±2.09 |
| *E. tonophilum* | 1.0±0.07 | 6.5±0.09 | 3.2±0.08 | 80.3±3.77 |
| Values represent the mean ± S.E No significant difference from control (LSD) | | | | |

**Table 4.** Effect of prolonged oral administration of the alcoholic extract of *Drechslera rostrata* and *Eurotium tonophilum* in a dose of 400 mg $kg^{-1}$ for 4 weeks on the serum concentration of creatinine and urea in rats before infection with *Leishmania major* (n = 6)

| Groups | Creatinine (mg/dL) | Urea (mg/dL) |
|---|---|---|
| Control | 0.86±0.04 | 21.3±1.22 |
| *D. rostrata* | 0.90±0.06 | 22.1±1.16 |
| *E. tonophilum* | 0.90±0.08 | 20.0±0.85 |
| Values represent the mean ± S.E. No significant difference from control (LSD) | | |

**Table 5:** Effect of prolonged oral administration of the alcoholic extract of *Drechslera rostrata* and *Eurotium tonophilum* in a dose of 100 mg $kg^{-1}$ for 4 weeks on the serum concentration of creatinine and urea in rats after infection and treatment of *Leishmania major* (n = 6)

| Treatment | Liver function (U/L) | | Kidney function (mg/dL) | |
|---|---|---|---|---|
| | AST(U/L) | ALT(U/L) | urea(mg/dL) | Creatinine(mg/dL) |
| Control | 65.11±2.62 | 144.62±5.39 | 33.16 ± 1.75 | 0.38 ± 0.05 |
| *D. rostrata* | 62.37±3.47 | 145.20±5.51 | 35.50 ± 1.83 | 0.40 ± 0.02 |
| *E. tonophilum* | 64.50±3.56 | 140.25±4.22 | 34.17 ± 1.78 | 0.35 ± 0.06 |
| Values represent the mean ± S.E. No significant difference from control (LSD). | | | | |

## Claims

1. Extract from a fungal material of *Drechslera rostrata* and/or *Eurotium tonophilum* for use as anti-*leishmania* agent against *Leishmania major.*

2. Extract for use according to claim 1, wherein the extract is an alcoholic extract, preferably is a methanolic extract.

3. Extract for use according to claim 1 or 2, wherein the fungal material comprises mycelia mat of *Drechslera rostrata* and/or *Eurotium tonophilum.*

4. Compound isolated from an extract from the fungal material of Drechslera rostrata and/or Eurothium tonophilum selected from hexan-1,2,3,4,5,6-hexol, diisooctylphthalate and/or 1,8-dihydroxy-3-methoxy-6-methyl antraquinone for use as *anti-leishmania* agent against *Leishmania major.*

5. Pharmaceutical composition comprising an extract for use according to claims 1 to 3 and/or at least one of the compounds for use according to claim 4 for use as *anti-leishmania* agent against *Leishmania major.*

6. Pharmaceutical composition according to claim 5, formulated for oral administration.

## Patentansprüche

1. Extrakt von einem Pilzmaterial von *Drechslera rostrata* und/oder *Eurotium tonophilum* für die Verwendung als ein *anti-leishmania* Wirkstoff gegen *Leishmania Major.*

2. Extrakt für die Verwendung nach Anspruch 1, wobei das Extrakt ein alkoholisches Extrakt, bevorzugt ein methanolisches Extrakt ist.

3. Extrakt für die Verwendung nach Anspruch 1 oder 2, wobei das Pilzmaterial eine Myzelmatte von *Drechslera rostrata* und/oder *Eurolium tonophilium* umfasst.

4. Verbindung isoliert aus einem Extrakt von dem Pilzmaterial von *Drechslera rostrata* und/oder *Eurotium tonophilum,* ausgewählt aus Hexan-1,2,3,4,5,6-hexol, Diisooctylphthalat und/oder 1,8-Dihydroxy-3-methoxy-6-methylanthrachinon, für die Verwendung als ein *anti-leishmania* Wirkstoff gegen *Leishmania Major.*

5. Pharmazeutische Zusammensetzung umfassend ein Extrakt für die Verwendung nach Ansprüchen 1 bis 3 und/oder mindestens eine der Verbindungen für die Verwendung nach Anspruch 4, für die Verwendung als ein anti-*leishmania* Wirkstoff gegen *Leishmania Major.*

6. Pharmazeutische Zusammensetzung nach Anspruch 5, welche für die orale Verabreichung formuliert ist.

## Revendications

1. Extrait issu d'un matériel fongique de *Drechslera rostrata* et/ou *Eurotium tonophilum* pour son utilisation en tant qu'agent anti-leishmanien contre *Leishmania major.*

2. Extrait pour son utilisation selon la revendication 1, dans lequel l'extrait est un extrait alcoolisé, de préférence un extrait méthanolique.

3. Extrait pour son utilisation selon la revendication 1 ou 2, dans lequel le matériel fongique comprend le feutrage mycélien de *Drechslera rostrata* et/ou *Eurotium tonophilum.*

4. Composé isolé à partir d'un extrait issu du matériel fongique de *Drechslera rostrata* et/ou *Eurotium tonophilum* choisi parmi l'hexan-1,2,3,4,5,6-hexol, le phtalate de diisooctyle et/ou la 1,8-dihydroxy-3-méthoxy-6-méthyl-antraquinone pour son utilisation en tant qu'agent anti-leishmanien contre *Leishmania major.*

5. Composition pharmaceutique comprenant un extrait pour son utilisation selon les revendications 1 à 3 et/ou au moins un des composés selon la revendication 4 pour leur utilisation en tant qu'agent anti-leishmanien contre *Leishmania major.*

6. Composition pharmaceutique selon la revendication 5, formulée pour une administration par voie orale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MAJUMDER et al.** *Indian Journal of Experimental Biology,* 1993, vol. 31, 888-90
- **CHATTERJEE ; GUPTA.** *Indian Drugs,* 1991, vol. 28, 224-227
- **JIANGTAO GAO et al.** *Med. Chem. Res.,* 2012, vol. 21, 3080-3086
- **DJALMA et al.** *J. Braz. Chem. Soc.,* 2009, vol. 20 (4), 644-651
- **BABSON et al.** *Clinical chemistry,* 1966, vol. 12, 482-490
- **WALTER ; GERARADE.** *Microchem. J.,* 1970, vol. 15, 231
- **HENARY et al.** *Clinical Chemistry Rpinciples and Techniques,* 1974
- **DOUMAS et al.** *Clin. Chim. Acta,* 1971, vol. 31, 87-96
- **MORIN ; PROX.** *Am. J. Clin. Phatol,* 1973, vol. 60 (5), 691
- **WILLS ; SAVORY.** *Annuals of Clinical and Laboratory Science,* 1981, vol. 11 (4), 292-299
- **KROLL et al.** *Clinical Chemistry,* 1987, vol. 33, 1129-1132